(19) Europäisches Patentamt / European Patent Office / Office européen des brevets

(11) **EP 4 435 673 A1**

(12) **EUROPEAN PATENT APPLICATION**
published in accordance with Art. 153(4) EPC

(43) Date of publication:
**25.09.2024 Bulletin 2024/39**

(51) International Patent Classification (IPC):
**G06N 3/09** *(2023.01)* **G06N 3/048** *(2023.01)*

(21) Application number: **23821470.4**

(22) Date of filing: **22.05.2023**

(86) International application number:
**PCT/JP2023/019012**

(87) International publication number:
**WO 2024/166405 (15.08.2024 Gazette 2024/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.02.2023 JP 2023017144**

(71) Applicant: **DIC CORPORATION**
**Itabashi-ku**
**Tokyo 174-8520 (JP)**

(72) Inventor: **NAGAO Atsushi**
**Takaishi-shi, Osaka 592-0001 (JP)**

(74) Representative: **TBK**
**Bavariaring 4-6**
**80336 München (DE)**

(54) **METHOD FOR ASSISTING IN EVALUATION OF LEARNING STEP IN PREDICTION MODEL, INFORMATION PROCESSING DEVICE, AND PROGRAM**

(57) To improve an evaluation support technology of a prediction model. A method for supporting evaluation of a learning process of a prediction model executed by an information processing device 10, the method includes: a step of training a neural network model including an input layer, an intermediate layer, and an output layer based on actual data including an explanatory factor and an objective factor; and a step of outputting statistical information related to input values input to the intermediate layer and the output layer of the neural network model.

FIG. 1

INFORMATION PROCESSING DEVICE — 10

CONTROL UNIT — 11

STORAGE UNIT — 12

INPUT UNIT — 13

OUTPUT UNIT — 14

## Description

Technical Field

The present disclosure relates to a method for supporting evaluation of a learning process of a prediction model, an information processing device, and a program. The present application claims priority based on Japanese Patent Application No. 2023-017144 filed in Japan on February 7, 2023, and the content thereof is incorporated herein.

Background Art

Conventionally, methods for performing prediction related to physical and chemical phenomena such as chemical reactions have been developed (for example, PTL 1).

Citation List

Patent Literature

PTL 1: WO 2003/026791

Summary of Invention

Technical Problem

The technique described in PTL 1 has described the use of modeling techniques such as neural networks, partial least squares, and principal component regression to optimize the control of reactor systems. However, a method for evaluating a learning process of a prediction model has not been considered in PTL 1, and there has been room for improvement in the evaluation support technology of the prediction model.
An object of the present disclosure made in view of such circumstances is to improve the evaluation support technology of the prediction model.

Solution to Problem

(1) A method according to one embodiment of the present disclosure is a method for supporting evaluation of a learning process of a prediction model executed by an information processing device, the method comprising:

a step of training a neural network model that comprises an input layer, an intermediate layer, and an output layer based on actual data that comprises an explanatory factor and an objective factor; and
a step of outputting statistical information related to input values input to the intermediate layer and the output layer of the neural network model.

(2) A method for supporting evaluation of a learning process of a prediction model according to one embodiment of the present disclosure is the method according to (1), in which the statistical information comprises a first frequency distribution related to an input value input to the intermediate layer and a second frequency distribution related to an input value input to the output layer.
(3) A method for supporting evaluation of a learning process of a prediction model according to one embodiment of the present disclosure is the method according to (2), further comprising:

a step of displaying the first frequency distribution together with an activation function related to the intermediate layer; and
a step of displaying the second frequency distribution together with an activation function related to the output layer.

(4) A method for supporting evaluation of the learning process of a prediction model according to one embodiment of the present disclosure is the method according to (2) or (3), further comprising:

a step of determining whether a first peak determined based on the first frequency distribution is within a first predetermined range determined based on an activation function related to the intermediate layer; and
a step of determining whether a second peak determined based on the second frequency distribution is within a second predetermined range determined based on an activation function related to the output layer.

(5) A method for supporting evaluation of the learning process of a prediction model according to one embodiment of the present disclosure is the method according to (4), in which the first predetermined range is a range where an output value of the activation function related to the intermediate layer is 0.01 or more and less than 0.99 or a range where a differential value of the activation function related to the intermediate layer is larger than 0, and the second predetermined range is a range where an output value of the activation function related to the output layer is 0.01 or more and less than 0.99 or a range where a differential value of the activation function related to the output layer is larger than 0.
(6) A method for supporting evaluation of the learning process of a prediction model according to one embodiment of the present disclosure is the method according to (5), in which the activation functions are sigmoid functions.
(7) A method for supporting evaluation of the learning process of a prediction model according to one embodiment of the present disclosure is the method according to any one of (1) to (3), in which an initial value of a weight coefficient of the neural network model is determined based on a first predetermined range determined based on an activation function related to the intermediate layer and a second predetermined range determined based on an activation function related to the output layer.

(8) An information processing apparatus according to one embodiment of the present disclosure is an information processing device supporting evaluation of a learning process of a prediction model, the information processing device comprising a control unit, in which

the control unit
trains a neural network model that comprises an input layer, an intermediate layer, and an output layer based on actual data that comprises an explanatory factor and an objective factor; and
outputs statistical information related to input values input to the intermediate layer and the output layer of the neural network model.

(9) A non-transitory computer-readable recording medium according to one embodiment of the present disclosure is a non-transitory computer-readable recording medium storing therein instructions, the instruction, when executed by a processor, causing the processor to execute:

training of a neural network model that comprises an input layer, an intermediate layer, and an output layer based on actual data that comprises an explanatory factor and an objective factor; and
output of statistical information related to input values input to the intermediate layer and the output layer of the neural network model.

Advantageous Effects of Invention

With the method, the information processing device, and the program according to one embodiment of the present disclosure, the evaluation support technology of the prediction model can be improved.

Brief Description of Drawings

FIG. 1 is a block diagram illustrating the schematic configuration of an information processing device according to the present embodiment.
FIG. 2 is a flowchart illustrating operations of the information processing device according to the present embodiment.

FIG. 3 is a conceptual diagram of a neural network model according to the present embodiment.
FIG. 4 is one example of statistical information related to input values input to an intermediate layer of the neural network model according to the present embodiment.
FIG. 5 is one example of statistical information related to an input value input to an output layer of the neural network model according to the present embodiment. Description of Embodiments

Hereinafter, a method, an information processing device, and a program for supporting the evaluation of the learning process of the prediction model according to the embodiment of the present disclosure will be described with reference to the drawings. A prediction target of a prediction model according to the present embodiment may be arbitrary. In other words, the technology according to the present embodiment can be used for the whole modeling using neural networks and the like. The prediction target of the prediction model according to the present embodiment includes, for example, chemical reactions of synthetic resins. Examples of the chemical reactions of the synthetic resins include polycondensation reactions and addition polymerization reactions. The polycondensation reactions include dehydration-condensation reactions. The chemical reactions of the prediction target of the prediction model according to the present embodiment are not limited to these, and the prediction target may be any chemical reaction other than those of the synthetic resins. Hereinafter, in the present embodiment, the case where the prediction target of the prediction model is chemical reactions of synthetic resins or the like will be described. The present invention, however, is not limited thereto.

In each of the drawings, identical or equivalent parts are assigned the same symbols. In the description of the present embodiment, descriptions of the identical or equivalent parts are omitted or simplified as appropriate.

First, the overview of the present embodiment will be described. The method according to the present embodiment is executed by an information processing device 10. The information processing device 10 trains a neural network model including an input layer, an intermediate layer, and an output layer based on actual data including an explanatory factor and an objective factor related to the chemical reaction. In other words, the prediction model of the target evaluated by the method according to the present embodiment is the neural network model. In the method according to the present embodiment, the information processing device 10 also outputs statistical information related to input values input to the intermediate layer and the output layer of the neural network model.

As described above, the method according to the present embodiment is characterized in that the statistical information related to the input values input to the intermediate layer and the output layer of the neural network model is output. As described later, the output of the statistical information enables the user to objectively evaluate the validity of a learning process of the prediction model predicting the chemical reaction. Therefore, the evaluation support technology according to the present embodiment can be used as an evaluation method that secures robustness of the prediction result even for unknown future data, which cannot be measured only by cross-validation, a common model evaluation method. Therefore, according the present embodiment, the evaluation support technology of the prediction model predicting the chemical reactions can be improved.

(Configuration of Information Processing Device)

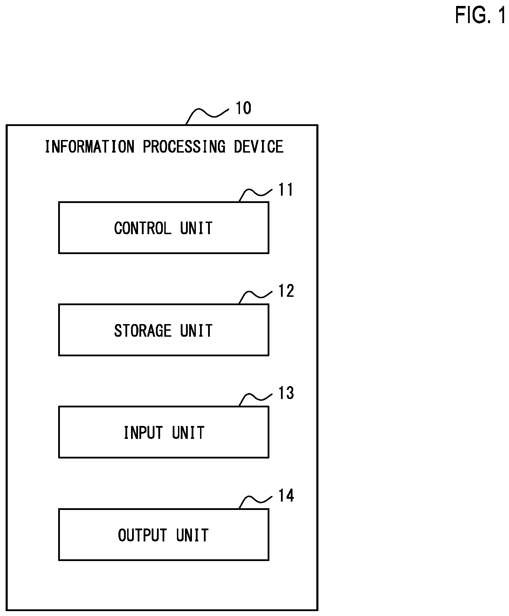

Subsequently, referring to FIG. 1, each configuration of the information processing device 10 will be described in detail. The information processing device 10 is an arbitrary device used by users. For example, personal computers, server computers, general-purpose electronic devices, or dedicated electronic devices can be employed as the information processing device 10.

As illustrated in FIG. 1, the information processing device 10 includes a control unit 11, a storage unit 12, an input unit 13, and an output unit 14.

The control unit 11 includes at least one processor, at least one dedicated circuit, or a combination thereof. The processor is a general-purpose processor such as a central processing unit (CPU) or a graphics processing unit (GPU), or a dedicated processor specialized for specific processing. The dedicated circuit is, for example, a field-programmable gate array (FPGA) or an application specific integrated circuit (ASIC). The control unit 11 executes processes associated with the operation of the information processing device 10 while controlling each part of the information processing device 10.

The storage unit 12 includes at least one semiconductor memory, at least one magnetic memory, at least one optical memory, or a combination of at least two of these memories. The semiconductor memory is, for example, a random-access memory (RAM) or a read-only memory (ROM). The RAM is, for example, a static random-access memory (SRAM) or a dynamic random-access memory (DRAM). The ROM is, for example, an electrically erasable programmable read-only memory (EEPROM). The storage unit 12 functions, for example, as a main memory device, an auxiliary memory device, or a cache memory. In the storage unit 12, data used in the operation of the information processing device 10 and data obtained by the operation of the information processing device 10 are stored.

The input unit 13 includes at least one interface for input. The interface for input is, for example, physical keys, capacitive keys, pointing devices, or touch screens integrated with displays. The interface for input may be, for example, a microphone that accepts voice input or a camera that accepts gesture input. The input unit 13 accepts operations to input data used in the operation of the information processing device 10. The input unit 13 may be connected to the information processing device 10 as an external input device instead of being provided in the information processing device 10. For example, any method such as universal serial bus (USB), high-definition multimedia interface (HDMI) (registered trademark), or Bluetooth (registered trademark) can be used as the connection method.

The output unit 14 includes at least one interface for output. The interface for output is, for example, a display that outputs information in the form of images. The display is, for example, a liquid crystal display (LCD) or an electroluminescence (EL) display. The output unit 14 displays and outputs data obtained by the operation of the information processing device 10. The output unit 14 may be connected to the information processing device 10 as an external output device instead of being provided in the information processing device 10. For example, any method such as USB, HDMI (registered trademark), or Bluetooth (registered trademark) can be used as the connection method.

The functions of the information processing device 10 are achieved by executing program according to the present embodiment on a processor corresponding to the information processing device 10. In other words, the functions of the information processing device 10 are achieved by the software. The program causes the computer to function as the information processing device 10 by causing the computer to execute the operations of the information processing device 10. In other words, the computer functions as the information processing device 10 by executing the operation of the information processing device 10 in accordance with the program.

In the present embodiment, the program can be recorded on a computer-readable recording medium. The computer-readable recording media include non-transient computer-readable media, for example, magnetic recording devices, optical discs, magneto-optical recording media, or semiconductor memories. Program distribution is performed by, for example, selling, assigning, or lending removal recording media such as a digital versatile disc (DVD) or a compact disc read only memory (CD-ROM) on which the program is recorded. Program distribution may also be done by storing the program in a storage on an external server and transmitting the program from the external server to other computers. The program may also be provided as a program product.

Some of or all of the functions of the information processing device 10 may be achieved by a dedicated circuit corresponding to the control unit 11. In other words, some or all of the functions of the information processing device 10 may be achieved by hardware.

In the present embodiment, the storage unit 12 stores therein, for example, actual data and prediction models. The actual data and the prediction model may be stored in an external device separate from the information processing device 10. In this case, the information processing device 10 may be equipped with an interface for external communication. The interface for communication may be either interface of a wired communication or interface of wireless communication. In the case of the wired communication, the interface for communication is, for example, a LAN interface or USB. In the case of the interface for wireless communication, the interface for communication is, for example, an interface compliant with mobile communication standards such as LTE, 4G, or 5G, or an interface compliant with short-range wireless communication such as Bluetooth (registered trademark). The interface for communication can receive data used in the operation of the information processing device 10 and can transmit data obtained by the operation of the information processing device 10.

(Operation of Information Processing Device)

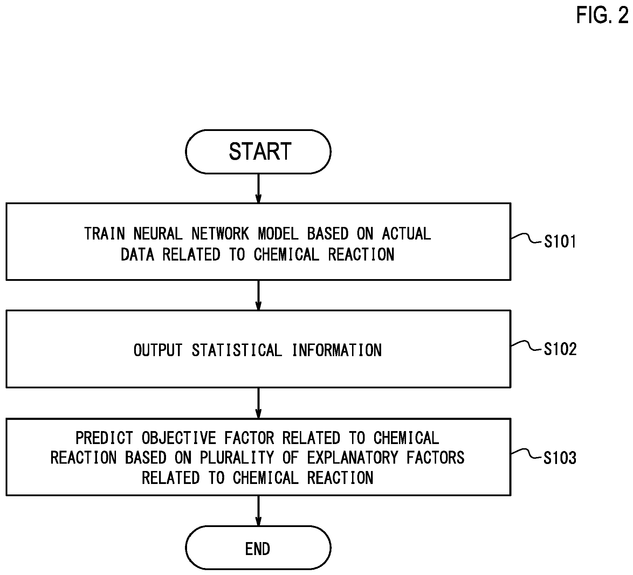

Subsequently, with reference to FIG. 2, the operation of the information processing device 10 according to the present embodiment will be described.

Step S101: The control unit 11 of the information processing device 10 trains a neural network model based on actual data related to a chemical reaction of a synthetic resin. The actual data include an explanatory factor and an objective factor related to the chemical reaction. Such explanatory factor and objective factor are appropriately selected depending on the target chemical reaction to be predicted. For example, in the case where the prediction related to the dehydration-condensation reaction is performed, the experimental data include the explanatory factors and the objective factor related to the dehydration-condensation reaction. For example, the explanatory factors may include feature values and the like related to the dehydration and temperature rising processes. The objective factor may also include a hydroxyl value, an acid value, and the like. In other words, the control unit 11 trains the neural network model using these explanatory factor and objective factor included in the actual data as learning data.

Any method can be employed to acquire the actual data. For example, the control unit 11 acquires the actual data from the storage unit 12. The control unit 11 may also acquire the actual data by accepting input of the actual data from the user by the input unit 13. Alternatively, the control unit 11 may acquire such actual data from an external device that stores the actual data through an interface for communication.

The neural network model trained based on the learning data is cross-validated. As a result of such cross-validation, in the case where an accuracy is within a practical range, the prediction related to the chemical reaction is performed using the neural network model.

Step S102: The control unit 11 outputs the statistical information related to the input values input to the intermediate layer and the output layer of the neural network model by the output unit 14.

Step S103: The control unit 11 predicts the objective factor related to the chemical reaction based on the explanatory factors related to the chemical reaction. For example, the control unit 11 may acquire the explanatory factors by accepting input of the explanatory factors from the user by the input unit 13. The control unit 11 may output the predicted objective factor as a prediction result by the output unit 14.

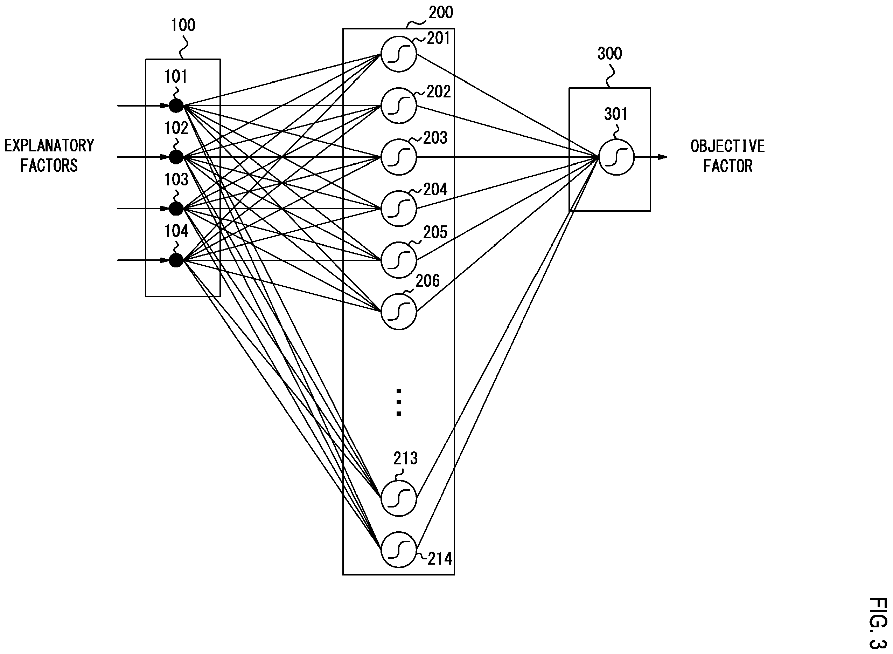

FIG. 3 is a conceptual diagram of the neural network model according to the present embodiment. The neural network model according to the present embodiment includes an input layer 100, an intermediate layer 200, and an output layer 300. The neural network model in the present embodiment is fully connected. In the present embodiment, the number of layers in the neural network model is, for example, 2. Such number of layers is the number of layers excluding the input layer. The number of layers of the neural network model according to the present embodiment is not limited to this number of layers, and may be three layers or more.

The input layer 100 includes a plurality of elements 101 to 104 (also referred to as input elements 101 to 104). In the neural network model illustrated in FIG. 3, the number of the input elements is 4. The input elements 101 to 104 are also referred to as the first to fourth elements, respectively. In the input elements 101 to 104, each of the explanatory factors is input. The number of input elements is not limited to this and may be less than 4 or 5 or more.

The intermediate layer 200 includes a plurality of elements 201 to 214 (also referred to as intermediate elements 201 to 214). In the neural network model illustrated in FIG. 3, the number of the intermediate elements is 14. The intermediate elements 201 to 214 are also referred to as the first to fourteenth elements, respectively. The number of intermediate elements is not limited to this, and may be less than 14 or 15 or more.

The output layer 300 includes an element 301 (an output element 301). In the neural network model illustrated in FIG. 3, the number of the output elements is 1. The output element 301 is also referred to as the first element The number of the output elements is not limited to this, and may be 2 or more.

The values input from the input elements 101 to 104 of the input layer 100 to the intermediate elements 201 to 214 of the intermediate layer 200 are converted in the intermediate layer 200 based on the activation function related to the intermediate layer 200. The converted values are output to the element 301 of the output layer 300. The activation function related to the intermediate layer 200 is, for example, a sigmoid function. The values input from the intermediate elements 201 to 214 of the intermediate layer 200 to the output element 301 of the output layer 300 are converted in the output layer 300 based on the activation function related to output layer 300 and output. The activation function related to the output layer 300 is, for example, the sigmoid function. Specifically, the activation functions related to the intermediate layer and the output layer are, for example, the respective sigmoid functions determined by the following Mathematical Formulas (1) and (2).

[Mathematical Formula 1]

$$f^1\left(u_j^1\right) = \frac{1}{1+e^{-a_1 u_j^1}} \quad (1)$$

$$f^2\left(u_j^2\right) = \frac{1}{1+e^{-a_2 u_j^2}} \quad (2)$$

Here, $f^1\left(u_j^1\right)$ is the activation function related to the intermediate layer 200, $\alpha_1$ is the coefficient of the activation function related to the intermediate layer 200, and $u_j^1$ is the input value input to the j-th element of the intermediate layer 200. $f^2\left(u_j^2\right)$ is the activation function related to the output layer 300, $\alpha_2$ is the coefficient of the activation function related to the output layer 300, and $u_j^2$ is the input value input to the j-th element of the output layer. $\alpha_1$ and $\alpha_2$ may be the same value or may be different values.

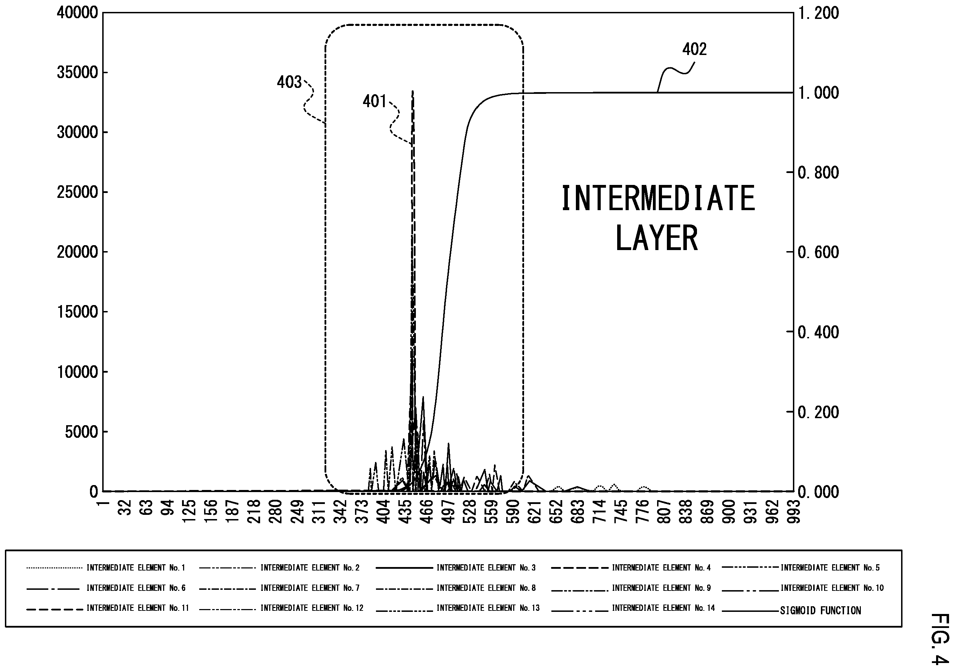

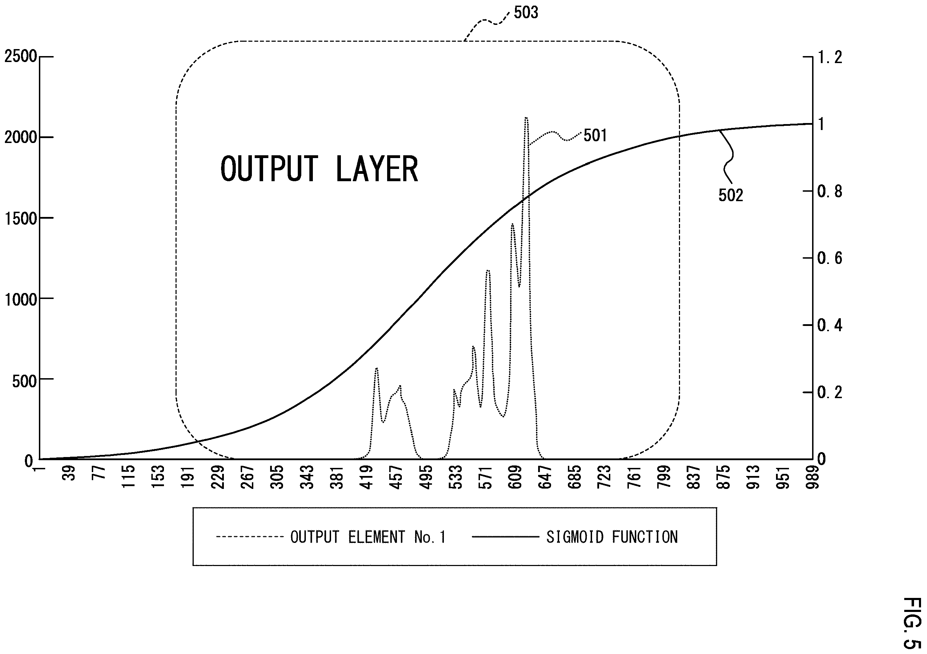

FIG. 4 and FIG. 5 illustrate specific examples of information including statistical information (hereinafter may also be referred to as output information) output by the output unit 14. FIG. 4 is one example of the output information including the statistical information related to the input values input to the intermediate layer. Such statistical information includes a first frequency distribution 401 related to the input values input to the intermediate layer. The first frequency distribution is information related to the frequency of the input values input to each intermediate element. The neural network according to the present embodiment includes the 14 intermediate elements 201 to 214. Therefore, the first frequency distribution according to the present embodiment includes information on the respective frequencies with respect to the intermediate elements. As illustrated in FIG. 4, the output information includes the first frequency distribution, whereby the peak values of the input values input to the intermediate elements 201 to 214 are visualized.

Here, the output information may include information on the activation function related to the intermediate layer. In other words, for example, the first frequency distribution 401 may be displayed together with the activation function related to the intermediate layer. The output information in FIG. 4 includes, for example, a graph 402 illustrating the activation function related to the intermediate layer. Here, the activation functions of all the 14 intermediate elements are the same in the present embodiment. Therefore, the activation function related to the intermediate layer is illustrated by the graph 402 alone. The output information includes the information on the activation function related to the intermediate layer, whereby the relationship between the activation function related to the intermediate layer and the first frequency distribution is visualized. More specifically, which region of the activation function related to the intermediate layer is used is visualized. For example, in the case where the activation function is the sigmoid function and the input values are concentrated in the linear parts at both ends of the function, the learning process using back-propagation (hereinafter may also be simply referred to as the learning process) is evaluated as not valid. In other words, in the case where the input values are concentrated in the linear parts at both ends of the function, it can be evaluated that there may be a failure to acquire nonlinearity in the learning process. On the other hand, in the case where the input values are concentrated in the central part (the curved part) of the sigmoid function, the learning process can be evaluated as valid.

The output information may include information related to a first predetermined range. The first predetermined range is a range where the output value of the activation function related to the intermediate layer is 0.01 or more and less than 0.99. The first predetermined range may be a range where the differential value of the activation function related to the intermediate layer is larger than 0. Alternatively, the first predetermined range may be a range where the differential value of the activation function related to the intermediate layer is larger than 0.001. The output information in FIG. 4 includes a rectangular box 403 representing the first predetermined range. As described above, the output information includes the information related to the first predetermined range, whereby the relationship between the first predetermined range and the first frequency distribution is visualized. For example, as illustrated in FIG. 4, when most of the input values represented by the first frequency distribution are within the first predetermined range, the learning process can be evaluated as valid. On the other hand, in the case where some of the input values represented by the first frequency distribution are not within the first predetermined range, the learning process can be evaluated as not valid.

FIG. 5 is one example of the output information including the statistical information related to the input values input to the output layer. Such statistical information includes a second frequency distribution 501 related to the input values input to the output layer. The second frequency distribution is information related to the frequencies of the respective input values input to the output elements. The neural network according to the present embodiment includes the one output element 301. Therefore, the second frequency distribution according to the present embodiment includes information on the frequency with respect to the output element 301. As illustrated in FIG. 5, the output information includes the second frequency distribution, whereby the peak values of the input values input to the output element 301 is visualized.

Here, the output information may include information on the activation function related to the output layer. In other words, for example, the second frequency distribution 501 may be displayed together with the activation function related to the output layer. The output information in FIG. 5 includes, for example, a graph 502 illustrating the activation function related to the output layer. The output information includes the information on the activation function related to the output layer, whereby the relationship between the activation function related to the output layer and the second frequency distribution is visualized. More specifically, which region of the activation function related to the output layer is used is visualized. For example, in the case where the activation function is a sigmoid function and the input values are concentrated in the linear parts at both ends of the function, the learning process can be evaluated as not valid. In other words, in the case where the input values are concentrated in the linear parts at both ends of the function, it can be evaluated that there may be a failure to acquire nonlinearity in the learning process. On the other hand, in the case where the input values are concentrated in the central part (the curved part) of the sigmoid function, the learning process can be evaluated as valid.

The output information may also include information related to a second predetermined range. The second predetermined range is a range where the output value of the activation function related to the output layer is 0.01 or more and less than 0.99. The second predetermined range may be a range where the differential value of the activation function related to the output layer is larger than 0. Alternatively, the second predetermined range may be a range where the differential value of the activation function related to the output layer is larger than 0.001. The output information in FIG. 5 includes a rectangular box 503 representing the second predetermined range. As described above, the output information includes the information related to the second predetermined range, whereby the relationship between the second predetermined range and the second frequency distribution is visualized. For example, as illustrated in FIG. 5, when most of the input values represented by the second frequency distribution are within the second predetermined range, the learning process can be evaluated as valid. On the other hand, in the case where some of the input values represented by the second frequency distribution are not within the second predetermined range, the learning process can be evaluated as not valid.

As described above, with the method according to the present embodiment, the validity of the learning process of the prediction model can be objectively evaluated by outputting the statistical information in relation to the learning process. Specifically, for example, the relationship between the first frequency distribution and the second frequency distribution and activation functions can be visualized by displaying them, whereby the validity of the learning process can be evaluated. Therefore, according the present embodiment, the evaluation support technology of the prediction model can be improved.

Here, in the present embodiment, hyperparameters may be adjusted in order to further optimize the learning process. For example, in the present embodiment, setting of the initial values of weight coefficients may be adjusted. For example, the initial values of the weight coefficients may be determined based on a first predetermined range determined based on the activation function related to the intermediate layer and a second predetermined range determined based on the activation function related to the output layer. As described above, the first predetermined range is, for example, a range where the output value of the activation function related to the intermediate layer is 0.01 or more and less than 0.99. The second predetermined range is, for example, a range where the output value of the activation function related to the output layer is 0.01 or more and less than 0.99. Specifically, for example, the weight coefficient between the input layer and the intermediate layer may be set to a random number from 0 or more and less than 0.1. The weight variable between the intermediate layer and the output layer may be set to a random number from 0 or more and less than 0.2. Setting the weight coefficient as described above allows respective starting points of learning to be adjusted so as to be in the first predetermined range and the second predetermined range. If the starting point is out of the range, it is desirable that the range of random numbers (the weight variables between the input layer and the intermediate layer are 0 or more and less than 0.1, and others) serving as the initial value of the weight coefficient be adjusted again.

In the present embodiment, the case where the intermediate layer is one layer is described. However, in the case where intermediate layers are two or more layers, the statistical information related to each intermediate layer may be output.

In FIG. 4, the plots of the peaks of the input values input to the intermediate elements may be highlighted. Highlighting such plots of peaks allows the relationship between the first predetermined range and the first frequency distribution to be more easily perceived. Similarly, in FIG. 5, the plots of the peaks of the input values input to the output element may be highlighted. Highlighting such plots of peaks allows the relationship between the second predetermined range and the second frequency distribution to be more easily perceived.

The determination of the validity of the learning process based on the first frequency distribution may be automated. For example, the control unit 11 may determine whether the first peak determined based on the first frequency distribution is within the first predetermined range. The control unit 11 may also output such a determination result from the output unit 14. The first peak is appropriately determined based on the first frequency distribution. For example, the first peak may be an average value, a median, or the like of the peaks in the frequency distribution of the input values of the intermediate elements. Alternatively, the first peak may be a set of peaks in the frequency distribution related to the input values of all intermediate elements. In other words, whether the peak in the frequency distribution related to the input values of all the intermediate elements is within the first predetermined range may be determined.

Similarly, the determination of the validity of the learning process based on the first frequency distribution may be automated. For example, the control unit 11 may determine whether the second peak determined based on the second frequency distribution is within the second predetermined range. The control unit 11 may also output such a determination result from the output unit 14. The second peak is appropriately determined based on the second frequency distribution. For example, the second peak may be an average value, a median, or the like of the peaks in the frequency distribution of the input values of the output elements. Alternatively, the second peak may be a set of peaks in the frequency distribution related to the input values of all intermediate elements. In other words, whether the peak in the frequency distribution related to the input values of all the intermediate elements is within the second predetermined range may be determined.

The case where the activation functions of the intermediate layer and the output layer are sigmoid functions is described in the present embodiment. However, the activation functions are not limited to the sigmoid functions. For example, the activation functions of the intermediate layer and the output layer may be functions such as a hyperbolic tangent function (tanh function) and a ramp functions (ReLU).

In this embodiment, the case where the prediction target is the chemical reaction of the synthetic resin and the like is described as one example. The prediction target, however, is not limited to this. The prediction target may be, for example, the prediction of a physical or chemical phenomenon such as a chemical reaction of any substance. The prediction target may not necessarily be a physical or chemical phenomenon and the like. In other words, the technology according to the present embodiment can be used for the whole modeling using neural networks and the like.

Although the present disclosure has been described based on the drawings and examples, it should be noted that those skilled in the art can easily make changes and modifications based on the present disclosure. Therefore, it should be noted that these changes and modifications are included within the scope of the present disclosure. For example, the functions and the like included in the units, steps, or the like can be rearranged so as not to be logically inconsistent, and a plurality of units, steps, or the like can be combined to one or divided.

Reference Signs List

10 information processing device
11 control unit
12 storage unit
13 input unit
14 output unit
100 input layer
200 intermediate layer
300 output layer
101 to 104, 201 to 214, and 301 element
401 first frequency distribution
501 second frequency distribution
402 and 502 graph
403 and 503 rectangular box

Claims

1. A method for supporting evaluation of a learning process of a prediction model executed by an information processing device, the method comprising:

   a step of training a neural network model that comprises an input layer, an intermediate layer, and an output layer based on actual data that comprises an explanatory factor and an objective factor; and
   a step of outputting statistical information related to input values input to the intermediate layer and the output layer of the neural network model.

2. The method according to claim 1, wherein the statistical information comprises a first frequency distribution related to an input value input to the intermediate layer and a second frequency distribution related to an input value input to the output layer.

3. The method according to claim 2, further comprising:

   a step of displaying the first frequency distribution together with an activation function related to the intermediate layer; and
   a step of displaying the second frequency distribution together with an activation function related to the output layer.

4. The method according to claim 2, further comprising:

   a step of determining whether a first peak determined based on the first frequency distribution is within a first predetermined range determined based on an activation function related to the intermediate layer; and
   a step of determining whether a second peak determined based on the second frequency distribution is within a second predetermined range determined based on an activation function related to the output layer.

5. The method according to claim 4, wherein the first predetermined range is a range where an output value of the activation function related to the intermediate layer is 0.01 or more and less than 0.99 or a range where a differential value of the activation function related to the intermediate layer is larger than 0, and the second predetermined range is a range where an output value of the activation function related to the output layer is 0.01 or more and less than 0.99 or a range where a differential value of the activation function related to the output layer is larger than 0.

6. The method according to claim 5, wherein the activation functions are sigmoid functions.

7. The method according to claim 1, wherein an initial value of a weight coefficient of the neural network model is determined based on a first predetermined range determined based on an activation function related to the intermediate layer and a second predetermined range determined based on an activation function related to the output layer.

8. An information processing device supporting evaluation of a learning process of a prediction model, the information processing device comprising a control unit, wherein

the control unit
trains a neural network model that comprises an input layer, an intermediate layer, and an output layer based on actual data that comprises an explanatory factor and an objective factor; and outputs statistical information related to input values input to the intermediate layer and the output layer of the neural network model.

9. A program supporting evaluation of a learning process of a prediction model that an information processing device executes, the program causing a computer to execute:

training of a neural network model that comprises an input layer, an intermediate layer, and an output layer based on actual data that comprises an explanatory factor and an objective factor; and output of statistical information related to input values input to the intermediate layer and the output layer of the neural network model.

FIG. 1

INFORMATION PROCESSING DEVICE — 10

CONTROL UNIT — 11

STORAGE UNIT — 12

INPUT UNIT — 13

OUTPUT UNIT — 14

FIG. 2

```
          ┌─────────────┐
          │    START    │
          └──────┬──────┘
                 │
                 ▼
  ┌────────────────────────────────────────┐
  │   TRAIN NEURAL NETWORK MODEL BASED ON   │───S101
  │   ACTUAL DATA RELATED TO CHEMICAL       │
  │   REACTION                              │
  └─────────────────┬──────────────────────┘
                    │
                    ▼
  ┌────────────────────────────────────────┐
  │      OUTPUT STATISTICAL INFORMATION     │───S102
  └─────────────────┬──────────────────────┘
                    │
                    ▼
  ┌────────────────────────────────────────┐
  │   PREDICT OBJECTIVE FACTOR RELATED TO   │───S103
  │   CHEMICAL REACTION BASED ON PLURALITY  │
  │   OF EXPLANATORY FACTORS RELATED TO     │
  │   CHEMICAL REACTION                     │
  └─────────────────┬──────────────────────┘
                    │
                    ▼
          ┌─────────────┐
          │     END     │
          └─────────────┘
```

FIG. 3

FIG. 4

FIG. 5

| INTERNATIONAL SEARCH REPORT | International application No. |
|---|---|
| | **PCT/JP2023/019012** |

**A. CLASSIFICATION OF SUBJECT MATTER**

***G06N 3/09***(2023.01)i; ***G06N 3/048***(2023.01)i
FI: G06N3/09; G06N3/048

According to International Patent Classification (IPC) or to both national classification and IPC

**B. FIELDS SEARCHED**

Minimum documentation searched (classification system followed by classification symbols)

G06N3/09; G06N3/048

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 3-118657 A (FUJITSU LTD) 21 May 1991 (1991-05-21)<br>p. 5, lower left column, line 8 to p. 9, lower left column, line 11 | 1-9 |
| A | JP 2019-070950 A (DENSO IT LAB INC) 09 May 2019 (2019-05-09)<br>paragraphs [0030]-[0141], fig. 10 | 1-9 |
| A | WO 2020/158760 A1 (TOKYO INST TECH) 06 August 2020 (2020-08-06)<br>paragraph [0057], fig. 3 | 1-9 |

☐ Further documents are listed in the continuation of Box C.　☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT

Information on patent family members

| International application No. |
|---|
| **PCT/JP2023/019012** |

| Patent document cited in search report | | | Publication date (day/month/year) | Patent family member(s) | Publication date (day/month/year) |
|---|---|---|---|---|---|
| JP | 3-118657 | A | 21 May 1991 | (Family: none) | |
| JP | 2019-070950 | A | 09 May 2019 | (Family: none) | |
| WO | 2020/158760 | A1 | 06 August 2020 | (Family: none) | |

Form PCT/ISA/210 (patent family annex) (January 2015)

**EP 4 435 673 A1**

**Patent documents cited in the description**

- JP 2023017144 A

- WO 2003026791 A